(19) Europäisches Patentamt
European Patent Office
Office européen des brevets

(11) Veröffentlichungsnummer: **0 004 978**
**B1**

(12) # EUROPÄISCHE PATENTSCHRIFT

(45) Veröffentlichungstag der Patentschrift: **29.07.81**

(51) Int. Cl.³: **C 11 B 9/00**, A 61 K 7/46

(21) Anmeldenummer: **79101216.4**

(22) Anmeldetag: **23.04.79**

(54) **Verwendung von 4,6,6-Trimethyltetrahydropyran-2-on bzw. eines Gemisches von 4,6,6-Trimethyltetrahydropyran-2-on mit 4,4,6-Trimethyltetrahydropyran-2-on im Gewichtsverhältnis 60:40 als Riechstoffe sowie diese enthaltende Riechstoffkompositionen.**

(30) Priorität: **26.04.78 DE 2818244**

(43) Veröffentlichungstag der Anmeldung:
**31.10.79 Patentblatt 79/22**

(45) Bekanntmachung des Hinweises auf die Patenterteilung:
**29.07.81 Patentblatt 81/30**

(84) Benannte Vertragsstaaten:
**BE CH FR GB IT NL**

(56) Entgegenhaltungen:
**DE - A - 2 756 772**
**GB - A - 1 179 669**

**Journal of Agricultural and Food Chemistry, June 1975 Washington D.C., USA, Vol. 23, Nr. 6, Seiten 1189—1195**
**A. O. PITTET and E. M. KLAIBER: "Synthesis and Flavor Properties of Some Alkyl-Substituted -Pyrone Derivatives"**

(73) Patentinhaber: **Henkel Kommanditgesellschaft auf Aktien**
**-Patentabteilung- Postfach 1100 Henkelstrasse 67**
**D-4000 Düsseldorf 1 (DE)**

(72) Erfinder: **Schaper, Ulf-Armin, Dr.**
**Nixenstrasse 17**
**D-4000 Düsseldorf 13 (DE)**
Erfinder: **Bruns, Klaus, Dr.**
**Notburgaweg 6**
**D-4150 Krefeld-Traar (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 004 978 B1**

Courier Press, Leamington Spa, England.

Verwendung von 4,6,6-Trimethyltetrahydropyran-2-on bzw. eines Gemisches von 4,6,6-Trimethyltetrahydropyran-2-on mit 4,4,6-Trimethyltetrahydropyran-2-on im Gewichts-Verhältnis 60:40 als Riechstoffe sowie diese enthaltende Riechstoffkompositionen

Es wurde gefunden, daß 4,6,6-Trimethyltetrahydropyran-2-on der Formel

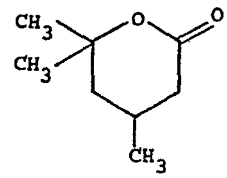

(I)

beziehungsweise ein Gemisch von (I) mit 4,6,6-Trimethyltetrahydropyran-2-on der Formel

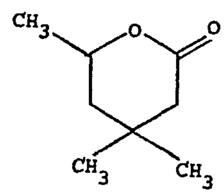

(II)

im Gewichts-Verhältnis (I) : (II) wie 60:40 in vorteilhafter Weise als Riechstoffe in Kompositionen zur Parfümierung technischer und kosmetischer Präparate verwendet werden können.

Die Herstellung der an sich literaturbekannten Verbindungen erfolgt mit guten Ausbeuten durch eine Oxidation des entsprechenden Trimethylcyclopentanons nach Baeyer-Villiger mittels einer Persäure. Als bevorzugtes Reagens dient Peressigsäure, es können aber auch andere organische oder anorganische Persäuren verwendet werden. Zu dem 4,6,6-Trimethyltetrahydropyran-2-on (I) (Beilstein E III/IV 17, 4227) gelangt man durch Einsatz des 2,2,4-Trimethylcyclopentanons als Ausgangsmaterial. Zur Herstellung eines technischen Produktes läßt sich ein unter der Bezeichnung Trimethylcyclopentanon (TMCP-on) handelsübliches Gemisch aus 60 Gewichtsteilen 2,2,4-Trimethylcyclopentanon und 40 Gewichtsteilen 2,4,4-Trimethylcyclopentanon verwenden. Aus diesem Gemisch resultiert dementsprechend ein 60:40-Gemisch von (I) und (II).

Das erfindungsgemäß zu verwendende 4,6,6-Trimethyltetrahydropyran-2-on (I) und das Gemisch von (I) mit 4,4,6-Trimethyltetrahydropyran-2-on (II) im Gewichts-Verhältnis (I) : (II) wie 60:40, sind wertvolle Riechstoffe mit charackteristischer Walnußnote. Sie lassen sich sehr gut zu neuartigen und interessanten Geruchsnuancen kombinieren. Das 4,6,6-Trimethyltetrahydropyran-2-on (I) ist ein interessanter und wertvoller Riechstoff mit einer mild-bitteren, typischen Walnußnote. Das Gemisch aus 60 Gewichtsteilen (I) und 40 Gewichtsteilen (II) besitzt noch die typische Walnuß-Note ähnlich (I). Von derartigen Riechstoffeigenschaften und parfümistischen Einsatzmöglichkeiten ist in der bekannten Vorliteratur nicht die Rede.

Das erfindungsgemäß als Riechstoff zu verwendende 4,6,6-Trimethyltetrahydropyran-2-on (I) bzw. das Gemisch von (I) mit 4,4,6-Trimethyltetrahydropyran-2-on (II) im Gewichts-Verhältnis (I) : (II) wie 60:40 können mit anderen Riechstoffen in den verschiedensten Mengenverhältnissen zu neuen Riechstoffkompositionen gemischt werden. Der Anteil des 4,6,6-Trimethyltetrahydropyran-2-ons (I) bzw. des 60/40-Gemisches von (I) und (II) in den Riechstoffkompositionen beträgt 1—50 Gewichtsprozent, bezogen auf die gesamte Komposition. Derartige Kompositionen können zur Parfümierung von Kosmetika wie Cremes, Lotionen, Aerosolen, Toiletteseifen, technischen Artikeln wie Wasch- und Reinigungsmitteln, Desinfektionsmitteln und Textilbehandlungsmitteln dienen. Für den Einsatz als alleinige Duftstoffe bieten sich auf Grund ihrer typischen Walnußnote kosmetische Lichtschutzpräparate wie Sonnenschutzcreme, Sonnenschutzöle und Sonnenschutzlotionen an.

Die nachfolgenden Beispiele sollen dem Gegenstand der Enfindung näher erläutern.

Beispiele

a) Herstellung der Ausgangsstoffe: 4,6,6-Trimethyltetrahydropyran-2-on (I)

3,84 g 2,2,4-Trimethylcyclopentanon wurden in 50 ml Chloroform gelöst und im Eisbad gekühlt. Bei 0°C wurde eine Lösung aus 0,2 g Natriumacetat in 11,4 g 40 %iger Peressigsäure zugegeben. Das Reaktionsgemisch wurde 18 Stunden lang bei Raumtemperatur gerührt, hernach mit Natriumhydrogencarbonatlösung neutralisiert, die organische Phase abgetrennt, getrocknet und eingeengt. Bei der Destillation wurden 3,4 g 4,6,6-Trimethyltetrahydropyran-2-on (I) mit folgenden Kennzahlen erhalten:

$Kp_{0,1\ mbar}$ 65°C Fp 29—30°C $n_D^{30}$ 1,4470

JR (Öl) $cm^{-1}$: 1730, 1455, 1373, 1275, 1215, 1110, 990, 918, 785.

| | | | | |
|---|---|---|---|---|
| NMR ($CDCl_3$) $\delta$ = | 1,03 ppm | d (6 Hz) | 3 H |
| | 1,4 | „ s | 3 H |
| | 1,42 | „ s | 3 H |
| | 1,6 | „ m | 2 H |
| | 2,05 | „ m | 1 H |
| | 2,6 | „ m | 2 H |

Die Geruchsnote war typisch Walnuß, fein mild-bitter.

60:40 — Gemisches von (I) und (II)

Bei der Umsetzung von 315 g handelsüblichem Trimethylcyclopentanon (TMCP-on der Firma Veba) entsprechend den vorstehenden Herstellungsangaben wurden 310 g eines Gemisches aus 4,6,6-Trimethyltetrahydropyran-2-on (I) und 4,4,6-Trimethyltetrahydropyran-2-on (II) im Gewichtsverhältnis von 60:40 erhalten. Das Gemisch besaß folgende Kennzahlen:

$Kp_{0,047}$ mbar 62°C $n_D^{20}$ 1,4484

Die Geruchsnote war typisch Walnuß, ähnlich dem Geruch von (I).

b) Riechstoffkompositionen:

Beispiel 1

Parfümierungsbeispiel einer Lichtschutz-Lotion

| | | |
|---|---|---|
| 4,6,6-Trimethyltetrahydroparan-2-on | 15 | Gewichtsteile |
| Tween 80® (=Polyoxyethylenesorbitanoleat) der Atlas Chemie GmbH | 60 | „ |
| Isopropylmyristat | 20 | „ |
| Propylenglykol | 20 | „ |
| Prosolal S8® (=Lichtschutzmittel) der Firma Haarmann & Reimer | 50 | „ |
| destilliertes Wasser | 835 | „ |

Beispiel 2

Riechstoffkomposition für Seifen und Waschmittel

| | | |
|---|---|---|
| 4,6,6-Trimethyltetrahydropyran-2-on | 150 | Gewichtsteile |
| p-tert.-Butylcyclohexylacetat | 80 | „ |
| Terpineol | 80 | „ |
| Isobornylacetat | 50 | „ |
| Benzylacetat | 50 | „ |
| Galaxolid® (=Moschusriechstoff) der IFF | 50 | „ |
| Lavandinöl, (=Öl aus Lavandula hybrida, Reverchon) | 50 | „ |

| | | |
|---|---|---|
| Jonon | 50 | Gewichtsteile |
| Cumarin | 40 | " |
| Benzylsalicylat | 40 | " |
| Zimtalkohol | 30 | " |
| Hydroxycitronellal | 30 | " |
| Petitgrainöl | 30 | " |
| Ylang-Öl | 30 | " |
| Galbanumöl synth. (der Firma Roue Bertrand Du Pont) | 20 | " |
| Cyclamenaldehyd | 20 | " |
| Dihydromyrcenol | 20 | " |
| Anisaldehyd | 20 | " |
| Diethylphthalat | 160 | " |

An die Stelle des in den vorstehenden Beispielen eingesetzten 4,6,6-Trimethyltetrahydropyran-2-on kann mit annähernd gleich gutem Erfolg das 60:40-Gemisch von 4,6,6- und 4,4,6-Trimethyltetrahydropyran-2-on treten.

**Patentansprüche**

1. Riechstoffkompositionen, dadurch gekennzeichnet, daß sie 4,6,6-Trimethyltetrahydropyran-2-on (I) bzw. ein Gemisch von (I) mit 4,4,6-Trimethyltetrahydropyranon-2-on (II) im Gewichts-Verhältnis (I) : (II) wie 60:40 in einer Menge 1 bis 50 Gewichtsprozent, bezogen auf die gesamte Komposition, enthalten.

2. Verwendung von 4,6,6-Trimethyltetrahydropyran-2-on der Formel

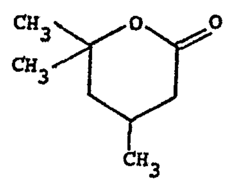

(I)

sowie eines Gemisches von (I) mit 4,4,6-Trimethyltetrahydropyran-2-on der Formel

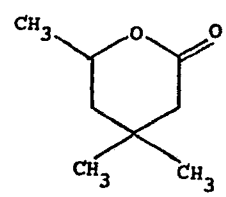

(II)

im Gewichts-Verhältnis (I) : (II) wie 60:40 als Riechstoffe.

**Claims**

1. Fragrance compositions, characterised in that they contain 4,6,6-trimethyl tetrahydropyran-2-one (I) or a mixture of (I) with 4,4,6-trimethyl tetrahydropyran-2-one (II) in a ratio by weight of (I) to (II) of 60:40 in a quantity of from 1 to 50% by weight, based on the composition as a whole.

2. The use of 4,6,6-trimethyl tetrahydropyran-2-one corresponding to the following formula

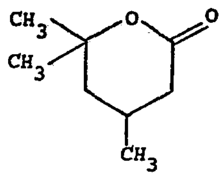

(I)

and a mixture of (I) with 4,4,6-trimethyl tetrahydropyran-2-one corresponding to the following formula

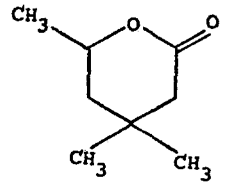

(II)

in a ratio by weight of (I) to (II) of 60:40 as fragrances.

**Revendications**

1. Compositions de substances odorantes, caractérisées en ce qu'elles contiennent de la 4,6,6-triméthyltétrahydropyrane-2-one (I) ou un mélange de (I) avec de la 4,4,6-triméthyltétrahydropyrane-2-one (II) dans le rapport pondéral (I):(II) de 60:40, en une quantité de 1 à 50% en poids par rapport à la composition totale.

2. Utilisation de la 4,6,6-triméthyltétrahydropyrane-2-one de formule:

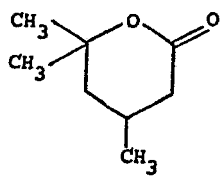

(I)

de même que d'un mélange de (1) avec de la 4,4,6-triméthyltétrahydropyrane-2-one de formule

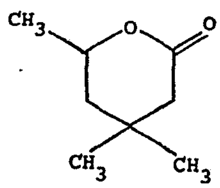

(II)

dans le rapport pondéral (I) : (II) de 60:40, en tant que substances odorantes.